# EUROPÄISCHE PATENTSCHRIFT

(11) **EP 3 713 542 B1**
(45) Veröffentlichungstag und Bekanntmachung des Hinweises auf die Patenterteilung: **23.03.2022**
(21) Anmeldenummer: 18812088.5
(22) Anmeldetag: 21.11.2018
(51) Int. Cl.: A61K 9/00, A61K 31/122

(54) **WASSERLÖSLICHE POLYMERKLEBSCHICHTEN**
WATER-SOLUBLE POLYMER ADHESIVE LAYERS
COUCHES ADHÉSIVES POLYMÈRES HYDROSOLUBLES

(30) Priorität: 21.11.2017 DE 102017127452
(43) Veröffentlichungstag der Anmeldung: 30.09.2020
(73) Patentinhaber: LTS Lohmann Therapie-Systeme AG, 56626 Andernach (DE)
(72) Erfinder: LINN, Michael, 55596 Waldböckelheim (DE); MÜLLER, Markus, 53840 Troisdorf (DE); BAUER, Marius, 56626 Andernach (DE)
(74) Vertreter: Held, Stephan
(86) Internationale Anmeldenummer: PCT/EP2018/082091
(87) Internationale Veröffentlichungsnummer: WO 2019/101798

(56) Entgegenhaltungen:
- EP-A1- 2 620 141
- US-A1- 2008 057 087
- VEERAN GOWDA KADAJJI ET AL: "Water Soluble Polymers for Pharmaceutical Applications", POLYMERS, Bd. 3, Nr. 4, 11. November 2011 (2011-11-11), Seiten 1972-2009, XP055555861, DOI: 10.3390/polym3041972
- SRINATH MUPPALANENI: "Polyvinyl Alcohol in Medicine and Pharmacy: A Perspective", JOURNAL OF DEVELOPING DRUGS, Bd. 02, Nr. 03, 1. Januar 2013 (2013-01-01), XP055555866, DOI: 10.4172/2329-6631.1000112

## Beschreibung

Die vorliegende Erfindung betrifft einen mehrschichtigen oralen Wirkstoff-Film, ein Verfahren zu dessen Herstellung und dessen Verwendung als Arzneimittel.

Orale Wirkstoff-Filme sind dünne, einen pharmazeutisch aktiven Wirkstoff enthaltende Filme, die direkt in den Mundraum gelegt oder an die Mundschleimhaut angelegt werden und sich dort auflösen. Diese dünnen oralen Wirkstoff-Filme können als ein- oder mehrschichtige Systeme aufgebaut sein. Der Wirkstoff kann in dem Film gelöst, emulgiert oder dispergiert werden.

Mehrschichtige orale Wirkstoff-Filme sind aus dem Stand der Technik bekannt.

So offenbart die WO 2011/134846 A1 mehrschichtige orale Wirkstoff-Filme, umfassend eine wirkstoffhaltige Schicht und eine Schicht enthaltend eine Substanz, die inkompatibel mit dem Wirkstoff in der wirkstoffhaltigen Schicht ist, wobei diese beiden Schichten durch eine weitere zwischen diesen beiden Schichten liegende Schutzschicht getrennt sind.

Die US 2013/0017235 A1 offenbart mehrschichtige orale Wirkstoff-Filme, bei denen eine wirkstoffhaltige Schicht von zwei wasserquellbaren Polymerschichten eingeschlossen wird.

Die aus dem Stand der Technik bekannten mehrschichtigen oralen Wirkstoff-Filme werden in der Regel gemäß einem Verfahren hergestellt, wodurch zunächst eine erste Schicht hergestellt wird und nach dem diese getrocknet wurde, eine zweite Schicht auf die erste Schicht aufgebracht wird. Nach dem Trocknen der zweiten Schicht auf der ersten Schicht kann dann gegebenenfalls eine dritte Schicht aufgebracht werden. Durch ein solches Verfahren können zwar vielschichtige Wirkstoff-Filme bereitgestellt werden, allerdings jeweils nur durch das Auflaminieren weiterer Schichten auf eine bereits bestehende Schicht. Dies hat den Nachteil, dass der pharmazeutisch aktive Wirkstoff aufgrund mehrfacher Beschichtung stärker thermisch und chemisch belastet wird. Zudem ist ein durch das Aufbringen einer Schicht auf eine bereits bestehen Schicht entstandener Verbund oft nicht stabil und kann leicht zerfallen. Des Weiteren pflanzen sich eventuelle Fehler in der Beschichtung, insbesondere fehlerhafte Flächengewichte, durch die nachfolgenden Beschichtungen fort.

Die Aufgabe der vorliegenden Erfindung besteht darin, vorstehend genannte Nachteile des Standes der Technik zu beheben. Insbesondere besteht die Aufgabe der vorliegenden Erfindung darin, einen mehrschichtigen oralen Wirkstoff-Film bereitzustellen, bei dem mehrere Schichten (es kann sich dabei um Schichten gleicher sowie unterschiedlicher Zusammensetzungen handeln) so verklebt werden, dass sich eventuelle Fehler bei der Herstellung einer Schicht nicht auf die anderen Schichten auswirken, wobei mindestens eine dieser Schichten mindestens einen pharmazeutisch aktiven Wirkstoff enthält, sodass ein fester Verbund dieser Schichten entsteht und der mindestens eine pharmazeutisch aktive Wirkstoff nicht mehrfach thermisch und/oder chemisch belastet wird. Vorzugsweise sollte sich der mehrschichtige orale Wirkstoff-Film bei der Anwendung in der Mundhöhle auflösen.

Obige Aufgabe wird durch einen mehrschichtigen oralen Wirkstoff-Film nach Anspruch 1 gelöst, der umfasst mindestens zwei nicht-klebende Schichten, die jeweils mindestens ein hydrophiles Polymer enthalten, sowie mindestens eine wasserlösliche Klebeschicht, die mindestens ein wasserlösliches Polymer und mindestens einen Weichmacher enthält, wobei mindestens eine der mindestens zwei nicht-klebende Schichten und/oder die mindestens eine wasserlösliche Klebeschicht einen pharmazeutisch aktiven Wirkstoff enthält, wobei die mindestens zwei nicht-klebenden Schichten und die mindestens eine wasserlösliche Klebeschicht so alternieren, dass die mindestens eine wasserlösliche Klebeschicht von beiden Seiten von mindestens einer nicht-klebenden Schicht bedeckt ist.

Insbesondere haben die Erfinder entdeckt, dass eine wasserlösliche Klebeschicht, die mindestens ein wasserlösliches Polymer und mindestens einen Weichmacher gemäß den Vorgaben in Anspruch 1 enthält, als dazwischenliegende wasserlösliche Klebeschicht zwei weitere Schichten, die an sich nicht klebrig sind, fest miteinander verkleben kann und somit der Aufbau von mehrschichtigen oralen Wirkstoff-Filmen ohne mehrfacher Übereinanderbeschichtung möglich ist.

Zudem haben die erfindungsgemäßen mehrschichtigen oralen Wirkstoff-Filme den Vorteil, dass jede Schicht separat herstellbar ist, damit gegebenenfalls auch gelagert werden kann, und somit leicht eine Vielzahl oraler Wirkstoff-Filme aus unterschiedlichen Kombinationen von Schichten hergestellt werden können.

Weiterhin besteht ein Vorteil darin, dass die Herstellung von Wirkstoff-Filmen mit hohen Flächengewichten, die deshalb eine lange Trocknungszeit aufweisen, vermieden werden kann. So kann beispielsweise ein Wirkstoff-Film mit einem Flächengewicht von 150 g/m² durch einen mehrschichtigen oralen Wirkstoff-Film ersetzt werden, bei dem zwei Schichten mit einem jeweiligen Flächengewicht von 75 g/m², durch eine dazwischenliegende Klebeschicht miteinander verklebt werden. Dies ist vorteilhaft, da die Trocknungszeit einer Schicht mit einem Flächengewicht von 75 g/m² deutlich kürzer ist.

Unter Klebeschicht wird hier eine Schicht verstanden, die als Klebstoff, wie in der DIN EN 923:2016-03 definiert, wirken kann. Eine nicht-klebende Schicht kann demnach nicht als Klebstoff wie vorstehend definiert wirken.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung dafür ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Ein hydrophiles Polymer ist ein Polymer, das polare oder geladene Gruppen enthält. Diese Gruppen können nicht-ionisch, anionisch, kationisch oder zwitterionisch sein. Hydrophile Polymere sind in der Regel wasserlöslich, können aber auch schlecht oder unlöslich in Wasser sein.

Weichmacher sind flüssige oder feste, indifferente organische Substanzen, vorzugsweise mit geringem Dampfdruck, welche ohne chemische Reaktion, vorzugsweise durch ihr Löse- und Quellvermögen, unter Umständen aber auch ohne ein solches, mit hochpolymeren Stoffen in physikalische Wechselwirkung treten und mit diesen ein homogenes System bilden können. Weichmacher verleihen den mit ihnen hergestellten Gebilden bzw. Überzügen bestimmte angestrebte physikalische Eigenschaften, wie z. B. erniedrigte Einfriertemperatur, erhöhtes Formveränderungsvermögen, erhöhte elastische Eigenschaften, verringerte Härte und gegebenenfalls gesteigertes Haftvermögen. Sie gehören zu den Kunststoffadditiven.

In dem erfindungsgemäßen mehrschichtigen oralen Wirkstoff-Film alternieren die nicht-klebenden Schichten und die wasserlösliche Klebeschicht bzw. die wasserlöslichen Klebeschichten derart, dass immer eine wasserlösliche Klebeschicht zwischen zwei nicht-klebenden Schichten vorliegt. Dabei können die nicht-klebenden Schichten die jeweils äußersten Schichten bilden, damit der erfindungsgemäße mehrschichtige orale Wirkstoff-Film an seinen Außenseiten nicht klebrig ist. Mit dieser Aufbauweise können prinzipiell orale Wirkstoff-Filme mit beliebig vielen Schichten bereitgestellt werden.

Bevorzugt umfasst der erfindungsgemäße mehrschichtige orale Wirkstoff-Film aber insgesamt drei Schichten, zwei nicht-klebende Schichten mit einer dazwischenliegenden wasserlöslichen Klebeschicht.

Es sind auch Ausführungsformen des erfindungsgemäßen mehrschichtigen oralen Wirkstoff-Films denkbar, bei denen eine der äußersten nicht-klebenden Schichten durch einen Kunststoff-Film, wie beispielsweise einen Film aus Polyethylenterephthalat, Polyethylen, Polybutylen, Polyurethan, Polyester, wasserunlöslicher Cellulose, wie Ethylcellulose etc., ersetzt wird.

Der pharmazeutisch aktive Wirkstoff kann in dem erfindungsgemäßen mehrschichtigen oralen Wirkstoff-Film grundsätzlich in jeder der Schichten des erfindungsgemäßen mehrschichtigen oralen Wirkstoff-Films enthalten sein. Dabei ist grundsätzlich die Kompatibilität des jeweiligen Wirkstoffs mit dem Material aus dem die jeweilige Schicht besteht zu berücksichtigen. Es ist jedoch auch die Kompatibilität des Wirkstoffs mit dem Material aus dem die übrigen Schichten bestehen zu berücksichtigen, da eine Migration des Wirkstoffs in dem mehrschichtigen System nicht ausgeschlossen werden kann.

Der erfindungsgemäße mehrschichtige orale Wirkstoff-Film ist nicht darauf beschränkt, dass nur ein pharmazeutisch aktiver Wirkstoff enthalten ist. So sind mehrschichtige orale Wirkstoff-Filme denkbar, bei denen in verschiedenen Schichten verschiedene pharmazeutisch aktive Wirkstoffe enthalten sind. Eine einzelne Schicht kann auch mehr als einen pharmazeutisch aktiven Wirkstoff enthalten.

Die vorliegende Erfindung ist besonders vorteilhaft bezüglich eines mehrschichtigen oralen Wirkstoff-Films, der in verschiedenen Schichten verschiedene Wirkstoffe enthält. Erfindungsgemäß können diese verschiedenen Schichten alle separat hergestellt werden und dann gemäß eines Baukastenprinzips durch die dazwischenliegenden Klebeschichten in beliebigen Kombinationen miteinander verklebt werden. Da auch die mindestens eine wasserlösliche Klebschicht, d.h. auch mehrere wasserlösliche Klebstoffschichten, pharmazeutisch aktive Wirkstoffe beinhalten kann bzw. können, kann die Anzahl an möglichen Kombinationen weiter erhöht werden. Somit sind auch komplexe mehrschichtige orale Wirkstoff-Filme leicht zugänglich.

Bevorzugt ist ein mehrschichtiger oraler Wirkstoff-Film, der mindestens einen pharmazeutisch aktiven Wirkstoff in mindestens einer der mindestens zwei nicht-klebenden Schichten enthält.

Außerdem bevorzugt ist ein mehrschichtiger oraler Wirkstoff-Film, der mindestens einen pharmazeutisch aktiven Wirkstoff in der mindestens einen wasserlöslichen Klebeschicht enthält.

Der mindestens eine pharmazeutisch aktive Wirkstoff kann aber auch in jeder anderen Schicht bzw. jeden andere Schichten und in beliebigen Kombinationen von Schichten vorhanden sein.

Generell kann in dem erfindungsgemäßen mehrschichtigen oralen Wirkstoff-Film jeder pharmazeutisch aktive Wirkstoff enthalten sein, der zur transmukosalen oder oralen Verabreichung geeignet ist.

Bevorzugt handelt es sich bei dem mindestens einen pharmazeutisch aktiven Wirkstoff um Idebenon.

Der erfindungsgemäße mehrschichtige orale Wirkstoff-Film zeichnet sich bevorzugt dadurch aus, dass das mindestens eine hydrophile Polymer in den mindestens zwei nicht-klebenden Schichten Polyvinylalkohol, Polyethylenoxid, Polyvinylpyrrolidon oder Copolymere davon oder ein Polyvinylcaprolactam-/Polyvinylacetat-/Polyethylenglykol-Copolymer (Soluplus) oder ein Vinylpyrrolidon-/Vinylacetat-Copolymer (Kollidon VA 64) oder ein Cellulosederivat, wie Hydroxypropylcellulose oder Hydroxypropylmethylcellulose, Stärke oder ein Stärkederivat, Schellack, Alginsäure, Galactomannan, Carrageen und andere pflanzliche Gummen, Pullulan, Xanthan, Pektin und anderen Glucanen, Dextran, Poly(meth)acrylaten, Polyalkylenglykolen, Carboxyvinylpolymeren und/oder Copolymeren davon, umfasst.

In einer besonders bevorzugten Ausführungsform umfasst das mindestens eine hydrophile Polymer Polyvinylalkohol, Polyvinylpyrrolidon und/oder Copolymere davon, Schellack oder ein Polyvinylcaprolactam-/Polyvinylacetat-/Polyethylenglykol-Copolymer oder ein Vinylpyrrolidon-/Vinylacetat-Copolymer oder ein Cellulosederivat, wie Hydroxypropylcellulose.

In einer besonders bevorzugten Ausführungsform ist der erfindungsgemäße orale Dünnfilm vorzugsweise dadurch gekennzeichnet, dass die mindestens zwei nicht-klebenden Schichten jeweils ein unterschiedliches hydrophiles Polymer ausgewählt aus der Gruppe, bestehend aus Polyvinylalkohol, Polyethylenoxid, Polyvinylpyrrolidon oder Copolymeren davon, Polyvinylcaprolactam-/Polyvinylacetat-/Polyethylenglykol-Copolymer, Vinylpyrrolidon-/Vinylacetat-Copolymer, einem Cellulosederivat, wie Hydroxypropylcellulose oder Hydroxypropymethyllcellulose, Stärke oder einem Stärkederivat, Schellack, Alginsäure, Galactomannan, Carrageen und andere pflanzliche Gummen, Pullulan, Xanthan, Pektin und anderen Glucanen, Dextran, Poly(meth)acrylate, Polyalkylenglykolen, Carboxyvinylpolymeren und/oder Copolymeren davon, umfassen.

Diese hydrophilen Polymere haben den Vorteil, dass sie mit einer Vielzahl von pharmazeutisch aktiven Wirkstoffen kompatibel und zudem weitgehend unbedenklich für einen Patienten sind, zu dessen Behandlung der erfindungsgemäße mehrschichtige oralen Wirkstoff-Film eingesetzt wird.

Diese hydrophilen Polymere haben außerdem den Vorteil, dass sie getrocknet einen dünnen stabilen Film bilden, der sich bei Applikation auf der Schleimhaut oder im Mundraum auflöst und damit den Wirkstoff freigibt. Dies hat den Vorteil einer schnellen Verfügbarkeit des Wirkstoffs sowie einer rückstandlosen Darreichung des Wirkstoffes.

Bei dem mindestens einen hydrophilen Polymer in den mindestens zwei nicht-klebenden Schichten kann es sich jeweils um das gleiche hydrophile Polymer handeln, es kann aber auch in jeder Schicht ein unterschiedliches hydrophiles Polymer vorliegen.

Der erfindungsgemäße mehrschichtige orale Wirkstoff-Film ist dadurch gekennzeichnet, dass das mindestens eine wasserlösliche Polymer in der mindestens einen wasserlöslichen Klebeschicht Schellack, ein Vinylpyrrolidon-/Vinylacetat-Copolymer, ein Polyvinylcaprolactam-/Polyvinylacetat-/Polyethyleneglykol-Copolymer, Hydroxypropylcellulose oder Hydroxypropylmethylcellulose und/oder Polyvinylpyrrolidon umfasst.

Der erfindungsgemäße mehrschichtige oralen Wirkstoff-Film umfasst außerdem als Weichmacher in der mindestens einen wasserlöslichen Klebeschicht Glycerin, Polyethylenglykol, insbesondere Polyethylenglykol 200, Sorbitol und/oder Tributylcitrat.

Besonders bevorzugt umfasst der mindestens eine Weichmacher in der mindestens einen wasserlöslichen Klebeschicht Glycerin, Polyethylenglykol 200 und/oder Tributylcitrat.

Durch den Einsatz, vorzugsweise als Mischung, mindestens eines wasserlöslichen Polymers und mindestens eines Weichmachers, entsteht eine klebrige Mischung, die bevorzugt nach Trocknen als Klebeschicht in dem erfindungsgemäßen mehrschichtigen oralen Wirkstoff-Film eingesetzt werden kann.

Das Gewichtsverhältnis des mindestens einen wasserlöslichen Polymers zu dem mindestens einen Weichmacher in der mindestens einen wasserlöslichen Klebeschicht beträgt etwa 80 bis 50 zu etwa 20 bis 50, bevorzugt etwa 80 bis 60 zu etwa 20 bis 40, noch bevorzugter etwa 80 bis 68 zu etwa 20 zu 32 und am bevorzugtesten etwa 80 bis 70 zu etwa 20 bis 30.

Wird zu wenig oder zu viel Weichmacher eingesetzt, so ist die Mischung entweder nicht klebrig oder ein verarbeitbarer Masseansatz kann von vorneherein nicht bereitgestellt werden.

Der mindestens eine pharmazeutisch aktive Wirkstoff ist grundsätzlich mindestens in einer pharmazeutisch wirksamen Menge in mindestens einer der Schichten des erfindungsgemäßen mehrschichtigen oralen Wirkstoff-Films enthalten.

Bevorzugt ist der mindestens eine pharmazeutisch aktive Wirkstoff in mindestens einer der mindestens zwei nicht-klebenden Schichten und/oder in der mindestens einen wasserlöslichen Klebeschicht vorhanden. Besonders bevorzugt ist es, wenn der pharmazeutisch aktive Wirkstoff jeweils in einer Menge von bis zu 75 Gew.-%, bevorzugt in einer Menge von 0,01 bis 75 Gew.-%, bezogen auf das Gesamtgewicht der jeweiligen Schicht, vorliegt.

Der erfindungsgemäße mehrschichtige orale Wirkstoff-Film ist weiterhin dadurch gekennzeichnet, dass die mindestens zwei nicht-klebenden Schichten jeweils ein Flächengewicht von 20 bis 200 g/m² aufweisen. Ist das Flächengewicht höher, so hat das den Nachteil besonders langer Trocknungszeiten, was wirtschaftlich unvorteilhaft ist. Ein Film mit einem geringeren Flächengewicht lässt sich nur schwer verarbeiten und ist damit nicht bevorzugt.

In den jeweiligen Schichten des erfindungsgemäßen mehrschichtigen oralen Wirkstoff-Films können außerdem übliche Zusatzstoffe, wie Permeationsverstärker, Antioxidantien, Geschmacksstoffe, geschmacksmaskierende Stoffe, Konservierungsstoffe, Farbstoffe, inerte Füllstoffe etc., enthalten sein.

Die vorliegende Erfindung betrifft auch ein Verfahren zur Herstellung eines mehrschichtigen oralen Wirkstoff-Films, wie vorstehend definiert, umfassend die Schritte
(a) Bereitstellen einer ersten nicht-klebenden Schicht, die mindestens ein hydrophiles Polymer enthält,
(b) Bereitstellen einer weiteren nicht-klebenden Schicht, die mindestens ein hydrophiles Polymer enthält,
(c) Bereitstellen einer wasserlöslichen Klebeschicht, die mindestens ein wasserlösliches Polymer und mindestens einen Weichmacher enthält,
(d) Aufbringen der Klebeschicht aus Schritt (c) auf eine der nicht-klebenden Schichten aus den Schritten (a) oder (b), um ein Laminat zu erhalten,
und
(e) Aufbringen der weiteren nicht-klebenden Schicht aus den Schritten (a) oder
(b) auf die wasserlösliche Klebeschicht des Laminats aus Schritt (d), so dass die wasserlösliche Klebeschicht von beiden Seiten von mindestens einer nicht-klebenden Schicht bedeckt ist, wobei mindestens eine der Schichten, die in den Schritten (a), (b) und (c) hergestellt werden, zusätzlich mindestens einen pharmazeutisch aktiven Wirkstoff enthält, und wobei das mindestens eine wasserlösliche Polymer und der mindestens eine Weichmacher in der wasserlöslichen Klebeschicht den vorstehenden Vorgaben entsprechen.

Es kann sich bei den Schichten a) und b) um Schichten verschiedener Zusammensetzung handeln. Es kann sich bei den Schichten a) und b) aber auch um Schichten gleicher Zusammensetzung handeln. Auch kann es sich bei den Schichten a) und b) um Schichten handeln, die aus der gleichen Ausgangsschicht erhalten wurden und dann gemäß dem obigen Verfahren miteinander verklebt wurden.

Umfasst der mehrschichtige orale Wirkstoff-Film insgesamt mehr als drei Schichten, so wird das alternierende Aufbringen von Klebeschichten und nicht-klebenden Schichten solange fortgesetzt, bis die gewünschte Schichtanzahl erreicht ist, wobei jeweils zwei nicht klebende Schichten die äußersten Schichten des mehrschichtigen oralen Wirkstoff-Films bilden.

Die vorliegende Erfindung betrifft außerdem einen mehrschichtigen oralen Wirkstoff-Film, erhältlich nach dem vorstehend geschilderten Verfahren.

Die vorliegende Erfindung betrifft weiterhin einen mehrschichtigen oralen Wirkstoff-Film wie vorstehend beschrieben oder erhältlich nach dem vorstehend geschilderten Verfahren zur Verwendung als Arzneimittel.

Die Erfindung wird nachfolgend anhand von nicht beschränkenden Beispielen erläutert.

### Beispiel 1:

Es wurden die in Tabelle 1 aufgeführten Mischungen eines wasserlöslichen Polymers und eines Weichmachers haptisch durch Aufdrücken mit dem Finger auf ihre Klebrigkeit getestet.

**Tabelle 1: Beispielformulierungen**

| Polymer | % Polymer | Weichmacher | % Weichmacher | Beurteilung |
|---|---|---|---|---|
| Soluplus | 80 | PEG 200 | 20 | Klebriq |
| Soluplus | 70 | PEG 200 | 30 | Klebriq |
| Soluplus | 80 | Glycerin | 20 | Klebriq |
| Soluplus | 90 | PEG 200 | 10 | Nicht klebriq |
| Soluplus | 90 | Glycerin | 10 | Nicht klebriq |
| Soluplus | 90 | Tributylcitrat | 10 | Nicht klebriq |
| PVP 30 | 80 | PEG 200 | 20 | Klebriq |
| PVP 30 | 70 | PEG 200 | 30 | Klebriq |
| PVP 30 | 80 | Glycerin | 20 | Klebriq |
| PVP 30 | 90 | PEG 200 | 10 | Nicht klebriq |
| PVP 30 | 90 | Glycerin | 10 | Nicht klebriq |
| PVP 30 | 90 | Tributylcitrat | 10 | Nicht klebriq |
| Kollidon VA 64 | 70 | Glycerin | 30 | Klebriq |
| Kollidon VA 64 | 70 | PEG 200 | 30 | Klebriq |
| Kollidon VA 64 | 70 | Tributylcitrat | 30 | Klebriq |
| Kollidon VA 64 | 80 | PEG 200 | 20 | Klebriq |
| Kollidon VA 64 | 80 | Glycerin | 20 | Klebriq |
| Kollidon VA 64 | 40 | PEG 200 | 60 | Masseansatz nicht möglich |
| Kollidon VA 64 | 40 | Glycerin | 60 | Masseansatz nicht möglich |
| Kollidon VA 64 | 40 | Tributylcitrat | 60 | Masseansatz nicht möglich |
| Kollidon VA 64 | 85 | Glycerin | 15 | Nicht klebriq |
| Kollidon VA 64 | 85 | PEG 200 | 15 | Nicht klebriq |
| Kollidon VA 64 | 85 | Tributylcitrat | 15 | Nicht klebriq |
| Schellack | 70 | Glycerin | 30 | Klebriq |
| Schellack | 70 | PEG 200 | 30 | Klebriq |
| Schellack | 70 | Tributylcitrat | 30 | Klebriq |
| Schellack | 50 | Glycerin | 50 | Klebriq |
| | | | | |
| HPC | 70 | Glycerin | 30 | Klebriq |
| HPC | 70 | PEG 200 | 30 | Klebriq |
| HPC | 50 | PEG 200 | 50 | Klebriq |
| | | | | |

| | |
|---|---|
| Soluplus: | Polyvinylcaprolactam-/Polyvinylacetat-/Polyethylenglykol-Co-polymer (BASF, Deutschland) |
| PVP 30: | Polyvinylpyrrolidon |
| Kollidon VA 64: | Vinylpyrrolidon-/Vinylacetat-Copolymer |
| HPC: | Hydroxypropylcellulose |
| PEG 200: | Polyethylenglykol 200 |

### Beispiel 2:

Die Herstellung einer wirkstoffhaltigen Schicht, bestehend aus 25% Idebenon (mikronisiert) und 75% Polyvinylalkohol (PVA 4-88) mit einem Flächengewicht von 150 g/m², würde aufgrund der Verwendung von Wasser als Lösemittel (bedingt durch PVA) und einer Trockentemperatur von 45°C (bedingt durch den niedrigen Schmelzpunkt von Idebenon) bei einem einschichtigen Wirkstoff-Film in nicht kommerziell darstellbare Trockenzeiten resultieren.

Dieses Problem kann durch einen erfindungsgemäßen mehrschichtigen oralen Wirkstoff-Film gelöst werden. Die wirkstoffhaltige Schicht wird mit zwei jeweils 75 g/m²-Schichten hergestellt bzw. mit einer Schicht, die für die Verwendung als Ober- und Unterseite aufgeteilt wird, womit die Trocknungszeiten auf ein akzeptables Maß reduziert werden können.

Getrennt davon wird eine wasserlösliche Klebeschicht aus 80% Kollidon VA 64 und 20% PEG 200 hergestellt. Diese Schicht wird auf die wirkstoffhaltige Schicht aufgetragen. Diese Klebeschicht wird anschließend mit einer weiteren wirkstoffhaltigen Schicht abgedeckt.

Dadurch entsteht ein Gesamtlaminat, welches die gewünschte 150 g/m² wirkstoffhaltige Schicht (als 2x75 g/m²) enthält. Die Klebeschicht hat in diesem Fall ein Flächengewicht von 60 g/m².

**Tabelle 2: Zusammensetzung eines erfindungsgemäßen mehrschichtigen Wirkstoff-Films.**

| | Material | Funktion | Gew.-% | Gew.-% final |
|---|---|---|---|---|
| Wirkstoffhaltige nicht-klebende Schicht (2x75 g/m²) | PVA 4-88 | Hydrophiles Polymer | 75 | 53,57 |
| | Idebenon | Wirkstoff | 25 | 17,86 |
| Klebeschicht (60 g/m²) | Kollidon VA 64 | Wasserlösliches Polymer | 80 | 22,86 |
| | PEG 200 | Weichmacher | 20 | 5,71 |

| | |
|---|---|
| PVA 4-88: | Polyvinylalkohol |
| Kollidon VA 64: | Vinylpyrrolidon-/Vinylacetat-Copolymer |
| PEG 200: | Polyethylenglykol 200 |

## Patentansprüche

1. Mehrschichtiger oraler Wirkstoff-Film, umfassend mindestens zwei nicht-klebende Schichten, die jeweils mindestens ein hydrophiles Polymer enthalten, sowie mindestens eine wasserlösliche Klebeschicht, die mindestens ein wasserlösliches Polymer und mindestens einen Weichmacher enthält, wobei mindestens eine der mindestens zwei nicht-klebende Schichten und/oder die mindestens eine wasserlösliche Klebeschicht einen pharmazeutisch aktiven Wirkstoff enthält, **dadurch gekennzeichnet, dass** die mindestens zwei nicht-klebenden Schichten und die mindestens eine wasserlösliche Klebeschicht so alternieren, dass die mindestens eine wasserlösliche Klebeschicht von beiden Seiten von mindestens einer nicht-klebenden Schicht bedeckt ist, wobei die Klebschicht eine Schicht darstellt, die als Klebstoff, wie in DIN EN 923:2016-03 definiert, wirken kann, wobei das mindestens eine wasserlösliche Polymer in der mindestens einen wasserlöslichen Klebeschicht Schellack, ein Vinylpyrrolidon-/Vinylacetat-Copolymer, ein Polyvinylcaprolactam-/Polyvinylacetat-/Polyethyleneglykol-Copolymer, Hydroxypropylcellulose oder Hydroxypropylmethylcellulose und/oder Polyvinylpyrrolidon umfasst und der mindestens eine Weichmacher in der mindestens einen wasserlöslichen Klebeschicht Glycerin, Polyethylenglykol, insbesondere Polyethylenglykol 200, und/oder Tributylcitrat umfasst, und wobei das Gewichtsverhältnis des mindestens einen wasserlöslichen Polymers zu dem mindestens einen Weichmacher in der mindestens einen wasserlöslichen Klebeschicht etwa 80 bis 50 zu etwa 20 bis 50 beträgt.

2. Mehrschichtiger oraler Wirkstoff-Film gemäß Anspruch 1, **dadurch gekennzeichnet, dass** der mindestens eine pharmazeutisch aktive Wirkstoff in mindestens einer der mindestens zwei nicht-klebenden Schichten enthalten ist.

3. Mehrschichtiger oraler Wirkstoff-Film gemäß Anspruch 1, **dadurch gekennzeichnet, dass** der mindestens eine pharmazeutisch aktive Wirkstoff in der mindestens einen wasserlöslichen Klebeschicht enthalten ist.

4. Mehrschichtiger oraler Wirkstoff-Film gemäß irgendeinem der vorhergehenden Ansprüche, **dadurch gekennzeichnet, dass** der mindestens eine pharmazeutisch aktive Wirkstoff Idebenon ist.

5. Mehrschichtiger oraler Wirkstoff-Film gemäß irgendeinem der vorhergehenden Ansprüche, **dadurch gekennzeichnet, dass** das mindestens eine hydrophile Polymer in den mindestens zwei nicht-klebenden Schichten Polyvinylalkohol, Polyethylenoxid, Polyvinylpyrrolidon oder Copolymere davon oder ein Polyvinylcaprolactam-/Polyvinylacetat-/Polyethylenglykol-Copolymer oder ein Vinylpyrrolidon-/Vinylacetat-Copolymer oder ein Cellulosederivat, wie Hydroxypropylcellulose oder Hydroxypropymethyllcellulose, Stärke, Schellack, Alginsäure, Galactomannan, Carrageen und andere pflanzliche Gummen, Pullulan, Xanthan, Pektin und anderen Glucanen, Dextran, Poly(meth)acrylat, Polyalkylenglykol, Carboxyvinylpolymer und/oder Copolymere davon umfasst.

6. Mehrschichtiger oraler Wirkstoff-Film gemäß Anspruch 5, **dadurch gekennzeichnet, dass** die mindestens zwei nicht-klebenden Schichten jeweils ein unterschiedliches hydrophiles Polymer ausgewählt aus der Gruppe, bestehend aus Polyvinylalkohol, Polyethylenoxid, Polyvinylpyrrolidon oder Copolymeren davon, Polyvinylcaprolactam-/Polyvinylacetat-/Polyethylenglykol-Copolymer, Vinylpyrrolidon-/Vinylacetat-Copolymer, eiemn Cellulosederivat, wie Hydroxypropylcellulose oder Hydroxypropymethyllcellulose, Stärke, Schellack, Alginsäure, Galactomannan, Carrageen und andere pflanzliche Gummen, Pullulan, Xanthan, Pektin und anderen Glucanen, Dextran, Poly(meth)acrylaten, Polyalkylenglykolen, Carboxyvinylpolymeren und/oder Copolymeren davon, umfassen.

7. Mehrschichtiger oraler Wirkstoff-Film gemäß irgendeinem der vorhergehenden Ansprüche, **dadurch gekennzeichnet, dass** der mindestens eine pharmazeutisch aktive Wirkstoff in mindestens einer der mindestens zwei nicht-klebenden Schichten und/oder in der mindestens einen wasserlöslichen Klebeschicht jeweils in einer Menge von bis zu 75 Gew.-%, bezogen auf das Gesamtgewicht der jeweiligen Schicht, vorliegt.

8. Mehrschichtiger oraler Wirkstoff-Film gemäß Anspruch 1, **dadurch gekennzeichnet, dass** das Gewichtsverhältnis des mindestens einen wasserlöslichen Polymers zu dem mindestens einen Weichmacher in der mindestens einen wasserlöslichen Klebeschicht etwa 80 bis 60 zu etwa 20 bis 40 beträgt.

9. Mehrschichtiger oraler Wirkstoff-Film gemäß irgendeinem der vorhergehenden Ansprüche, **dadurch gekennzeichnet, dass** die mindestens zwei nicht-klebenden Schichten jeweils ein Flächengewicht von 20 bis 200 g/m² aufweisen.

10. Mehrschichtiger oraler Wirkstoff-Film gemäß irgendeinem der vorhergehenden Ansprüche, **dadurch gekennzeichnet, dass** die mindestens eine wasserlösliche Klebeschicht ein Flächengewicht von 20 bis 200 g/m² aufweist.

11. Verfahren zur Herstellung eines mehrschichtigen oralen Wirkstoff-Films gemäß irgendeinem der Ansprüche 1 bis 10, umfassend die Schritte
(a) Bereitstellen einer ersten nicht-klebenden Schicht, die mindestens ein hydrophiles Polymer enthält,
(b) Bereitstellen einer weiteren nicht-klebenden Schicht, die mindestens ein hydrophiles Polymer enthält,
(c) Bereitstellen einer wasserlöslichen Klebeschicht, die mindestens ein wasserlösliches Polymer und mindestens einen Weichmacher gemäß den Vorgaben in Anspruch 1 enthält,
(d) Aufbringen der Klebeschicht aus Schritt (c) auf eine der nicht-klebende Schichten aus den Schritten (a) oder (b), um ein Laminat zu erhalten, und
(e) Aufbringen der weiteren nicht-klebenden Schicht aus den Schritten (a) oder (b) auf die wasserlösliche Klebeschicht des Laminats aus Schritt (d), so dass die wasserlösliche Klebeschicht von beiden Seiten von mindestens einer nicht-klebenden Schicht bedeckt ist,
wobei mindestens eine der Schichten, die in den Schritten (a), (b) und (c) hergestellt werden, zusätzlich mindestens einen pharmazeutisch aktiven Wirkstoff enthält.

12. Mehrschichtiger oraler Wirkstoff-Film gemäß irgendeinem der Ansprüche 1 bis 10 zur Verwendung als Arzneimittel,

## Claims

1. A multi-layer oral active-substance film comprising at least two non-adhesive layers each containing at least one hydrophilic polymer, and at least one water-soluble adhesive layer containing at least one water-soluble polymer and at least one plasticiser, wherein at least one of the at least two non-adhesive layers and/or the at least one water-soluble adhesive layer contains a pharmaceutically active substance, **characterised in that** the at least two non-adhesive layers and the at least one water-soluble adhesive layer alternate in such a way that the at least one water-soluble adhesive layer is covered on both sides by at least one non-adhesive layer, wherein the adhesive layer is a layer that can act as adhesive, as defined in DIN EN 923:2016-03, wherein the at least one water-soluble polymer in the at least one water-soluble adhesive layer comprises shellac, a vinylpyrrolidone/vinyl acetate copolymer, a polyvinyl caprolactam/polyvinyl acetate/polyethylene glycol copolymer, hydroxypropyl cellulose or hydroxypropyl methylcellulose and/or polyvinylpyrrolidone and wherein the at least one plasticiser in the at least one water-soluble adhesive layer comprises glycerol, polyethylene glycol, especially polyethylene glycol 200, and/or tributyl citrate, and wherein the weight ratio of the at least one water-soluble polymer to the at least one plasticiser in the at least one water-soluble adhesive layer is approximately 80 to 50 to approximately 20 to 50.

2. The multi-layer oral active-substance film according to claim 1, **characterised in that** the at least one pharmaceutically active substance is contained in at least one of the at least two non-adhesive layers.

3. The multi-layer oral active-substance film according to claim 1, **characterised in that** the at least one pharmaceutically active substance is contained in the at least one water-soluble adhesive layer.

4. The multi-layer oral active-substance film according to any one of the preceding claims, **characterised in that** the at least one pharmaceutically active substance is idebenone.

5. The multi-layer oral active-substance film according to any one of the preceding claims, **characterised in that** the at least one hydrophilic polymer in the at least two non-adhesive layers comprises polyvinyl alcohol, polyethylene oxide, polyvinylpyrrolidone or copolymers thereof or a polyvinyl caprolactam/polyvinyl acetate/polyethylene glycol copolymer or a vinylpyrrolidone/vinyl acetate copolymer or a cellulose derivative, such as hydroxypropyl cellulose or hydroxypropyl methylcellulose, starch, shellac, alginic acid, galactomannan, carrageenan and other natural gums, pullulan, xanthan, pectin and other glucans, dextran, poly(meth)acrylate, polyalkylene glycol, carboxyvinyl polymer and/or copolymers thereof.

6. The multi-layer oral active-substance film according to claim 5, **characterised in that** the at least two non-adhesive layers each comprise a different hydrophilic polymer selected from the group consisting of polyvinyl alcohol, polyethylene oxide, polyvinylpyrrolidone or copolymers thereof, polyvinyl caprolactam/polyvinyl acetate/polyethylene glycol copolymer, vinylpyrrolidone/vinyl acetate copolymer, a cellulose derivative, such as hydroxypropyl cellulose or hydroxypropyl methylcellulose, starch, shellac, alginic acid, galactomannan, carrageenan and other natural gums, pullulan, xanthan, pectin and other glucans, dextran, poly(meth)acrylates, polyalkylene glycols, carboxyvinyl polymers and/or copolymers thereof.

7. The multi-layer oral active-substance film according to any one of the preceding claims, **characterised in that** the at least one pharmaceutically active substance in at least one of the at least two non-adhesive layers and/or in the at least one water-soluble adhesive layer is present in each case in an amount of up to 75 wt.%, in relation to the total weight of the layer in question.

8. The multi-layer oral active-substance film according to any one of the preceding claims, **characterised in that** the weight ratio of the at least one water-soluble polymer to the at least one plasticiser in the at least one water-soluble adhesive layer is approximately 80 to 60 to approximately 20 to 40.

9. The multi-layer oral active-substance film according to any one of the preceding claims, **characterised in that** the at least two non-adhesive layers each have an areal density of from 20 to 200 g/m².

10. The multi-layer oral active-substance film according to any one of the preceding claims, **characterised in that** the at least one water-soluble adhesive layer has an areal density of from 20 to 200 g/m².

11. A method for producing a multi-layer oral active-substance film according to any one of claims 1 to 10, comprising the following steps
(a) providing a first non-adhesive layer containing at least one hydrophilic polymer,
(b) providing a further non-adhesive layer containing at least one hydrophilic polymer,
(c) providing a water-soluble adhesive layer containing at least one water-soluble polymer and at least one plasticiser according to the requirements of claim 1,
(d) applying the adhesive layer from step (c) to one of the non-adhesive layers from steps (a) or (b) in order to obtain a laminate, and
(e) applying the further non-adhesive layer from steps (a) or (b) to the water-soluble adhesive layer of the laminate from step (d) so that the water-soluble adhesive layer is covered from both sides by at least one non-adhesive layer,
at least one of the layers produced in steps (a), (b) and (c) additionally containing at least one pharmaceutically active substance.

12. The multi-layer oral active-substance film according to any one of claims 1 to 10 for use as a medicinal product.

## Revendications

1. Film multicouche à principe actif d'hygiène bucco-dentaire, comprenant au moins deux couches non adhésives, qui contiennent respectivement au moins un polymère hydrophile, ainsi qu'au moins une couche adhésive hydrosoluble, qui contient au moins un polymère hydrosoluble et au moins un plastifiant, dans lequel au moins une des au moins deux couches non adhésives et/ou l'au moins une couche hydrosoluble contiennent un principe pharmaceutiquement actif, **caractérisé en ce que** les au moins deux couches non adhésives et l'au moins une couche adhésive hydrosoluble alternent de telle sorte que l'au moins une couche adhésive hydrosoluble est recouverte des deux côtés d'au moins une couche non adhésive, dans lequel la couche adhésive constitue une couche qui peut agir en tant que colle ainsi que cela est défini dans la norme DIN:EN 923:2016-03, dans lequel l'au moins un polymère hydrosoluble dans l'au moins une couche adhésive hydrosoluble comprend de la gomme-laque, un copolymère de vinylpyrrolidone/acétate de vinyle, un copolymère de polyvinylcaprolactame/acétate de polyvinyle/polyéthylène glycol, de l'hydroxypropylcellulose ou de l'hydroxypropylméthylcellulose et/ou de la polyvinylpyrrolidone et l'au moins un plastifiant dans l'au moins une couche adhésive hydrosoluble comprend de la glycérine, du polyéthylène glycol, en particulier du polyéthylène glycol 200, et/ou du citrate de tributyle, et dans lequel le rapport de poids entre l'au moins un polymère hydrosoluble et à l'au moins un plastifiant dans l'au moins une couche adhésive hydrosoluble va d'environ 80 à 50: environ 20 à 50.

2. Film multicouche à principe actif d'hygiène bucco-dentaire selon la revendication 1, caractérisé en en ce que l'au moins un principe pharmaceutiquement actif est contenu dans au moins une des au moins deux couches non adhésives.

3. Film multicouche à principe actif d'hygiène bucco-dentaire selon la revendication 1, **caractérisé en ce que** l'au moins un principe pharmaceutiquement actif est contenu dans l'au moins une couche adhésive hydrosoluble.

4. Film multicouche à principe actif d'hygiène bucco-dentaire selon l'une quelconque des revendications précédentes, **caractérisé en ce que** l'au moins un principe pharmaceutiquement actif est de l'idébénone.

5. Film multicouche à principe actif d'hygiène bucco-dentaire selon l'une quelconque des revendications précédentes, **caractérisé en ce que** l'au moins un polymère hydrophile dans les au moins deux couches non adhésives comprend de l'alcool polyvinylique, de l'oxyde de polyéthylène, de la polyvinylpyrrolidone ou des copolymères de ceux-ci ou un copolymère de polyvinylcaprolactame/acétate de polyvinyle/polyéthylène glycol ou un copolymère de vinylpyrrolidone/acétate de vinyle ou un dérivé de cellulose, comme de l'hydroxypropylcellulose ou de l'hydroxypropylméthylcellulose, de l'amidon, de la gomme-laque, de l'acide alginique, du galactomannane, du carraghénane et d'autres gommes végétales, du pullulan, de la xanthane, de la pectine et d'autres glucanes, du dextrane, du poly(méth)acrylate, du polyalkylène glycol, du polymère carboxyvinylique et/ou des copolymères de ceux-ci.

6. Film multicouche à principe actif d'hygiène bucco-dentaire selon la revendication 5, **caractérisé en ce que** les au moins deux couches non adhésives comprennent respectivement un polymère hydrophile différent choisi parmi le groupe constitué d'alcool polyvinylique, d'oxyde de polyéthylène, de polyvinylpyrrolidone ou de copolymères de ceux-ci, de copolymère de polyvinylcaprolactame/acétate de polyvinyle/polyéthylène glycol, de copolymère de vinylpyrrolidone/acétate de vinyle, d'un dérivé de cellulose, comme de l'hydroxypropylcellulose ou de l'hydroxypropylméthylcellulose, de l'amidon, de la gomme-laque, de l'acide alginique, du galactomannane, du carraghénane et d'autres gommes végétales, du pullulan, de la xanthane, de la pectine et d'autres glucanes, du dextrane, des poly(méth)acrylates, des polyalkylène glycols, des polymères carboxyvinyliques et/ou des copolymères de ceux-ci.

7. Film multicouche à principe actif d'hygiène bucco-dentaire selon l'une quelconque des revendications précédentes, **caractérisé en ce que** l'au moins un principe pharmaceutiquement actif dans au moins une des au moins deux couches non adhésives et/ou dans l'au moins une couche adhésive hydrosoluble est présent respectivement en une quantité allant jusqu'à 75 % en poids par rapport au poids total de la couche respective.

8. Film multicouche à principe actif d'hygiène bucco-dentaire selon la revendication 1, **caractérisé en ce que** le rapport de poids entre l'au moins un polymère hydrosoluble et l'au moins un plastifiant dans l'au moins une couche adhésive hydrosoluble est d'environ 80 à 60: environ 20 à 40.

9. Film multicouche à principe actif d'hygiène bucco-dentaire selon l'une quelconque des revendications précédentes, **caractérisé en ce que** les au moins deux couches non adhésives présentent respectivement un poids surfacique de 20 à 200 g/m².

10. Film multicouche à principe actif d'hygiène bucco-dentaire selon l'une quelconque des revendications précédentes, **caractérisé en ce que** l'au moins une couche adhésive hydrosoluble présente un poids surfacique de 20 à 200 g/m².

11. Procédé de fabrication d'un film multicouche à principe actif d'hygiène bucco-dentaire selon l'une quelconque des revendications 1 à 10, comprenant les étapes
(a) de fourniture d'une première couche non adhésive, qui contient au moins un polymère hydrophile,
(b) de fourniture d'une autre couche non adhésive, qui contient au moins un polymère hydrophile,
(c) de fourniture d'une couche adhésive hydrosoluble, qui contient au moins un polymère hydrosoluble et au moins un plastifiant selon les spécifications dans la revendication 1,
(d) d'application de la couche adhésive issue de l'étape (c) sur une des couches non adhésives issues des étapes (a) ou (b) pour obtenir un stratifié, et
(e) d'application de l'autre couche non adhésive issue des étapes (a) ou (b) sur la couche adhésive hydrosoluble du stratifié issu de l'étape (d) de telle sorte que la couche adhésive hydrosoluble est recouverte des deux côtés par au moins une couche non adhésive,
dans lequel au moins une des couches, qui sont fabriquées lors des étapes (a), (b) et (c), contient en supplément au moins un principe pharmaceutiquement actif.

12. Film multicouche à principe actif d'hygiène bucco-dentaire selon l'une quelconque des revendications 1 à 10 destiné à être utilisé en tant que médicament.
